(19) Europäisches Patentamt
European Patent Office
Office européen des brevets

(11) Publication number: **0 164 889**
**A2**

# (12) EUROPEAN PATENT APPLICATION

(21) Application number: 85303239.9

(22) Date of filing: 07.05.85

(51) Int. Cl.[4]: G 01 N 33/545
C 08 F 291/00, C 08 J 7/16

(30) Priority: 07.05.84 CA 453747
22.04.85 US 725708

(43) Date of publication of application:
18.12.85 Bulletin 85/51

(84) Designated Contracting States:
AT BE CH DE FR GB IT LI NL SE

(71) Applicant: HSC Research Development Corporation
555 University Avenue
Toronto Ontario M5G 1X8(CA)

(72) Inventor: Pollak, Alfred
135 Marlee Avenue, Apt.1400
Toronto Ontario, M6B 4G6(CA)

(74) Representative: Corin, Christopher John et al,
Mathisen Macara & Co. The Coach House 6-8 Swakeleys Road
Ickenham Uxbridge UB10 8BZ(GB)

## (54) Surface-treated polymeric substrates.

(57) A surface treated polymeric substrate is provided for use in immobilizing molecules containing available amino groups, such as antibodies and antigons, the substrate having at least one polymer bonded thereto, the polymer comprising a mixture of at least two monomers, one of which contains an active group available for bonding with the amino groups in the molecules to be immobilized whereby multiple bonding sites are dispersed on said polymer and spaced from said substrate. A process for the production of such a substrate is also provided.

EP 0 164 889 A2

./...

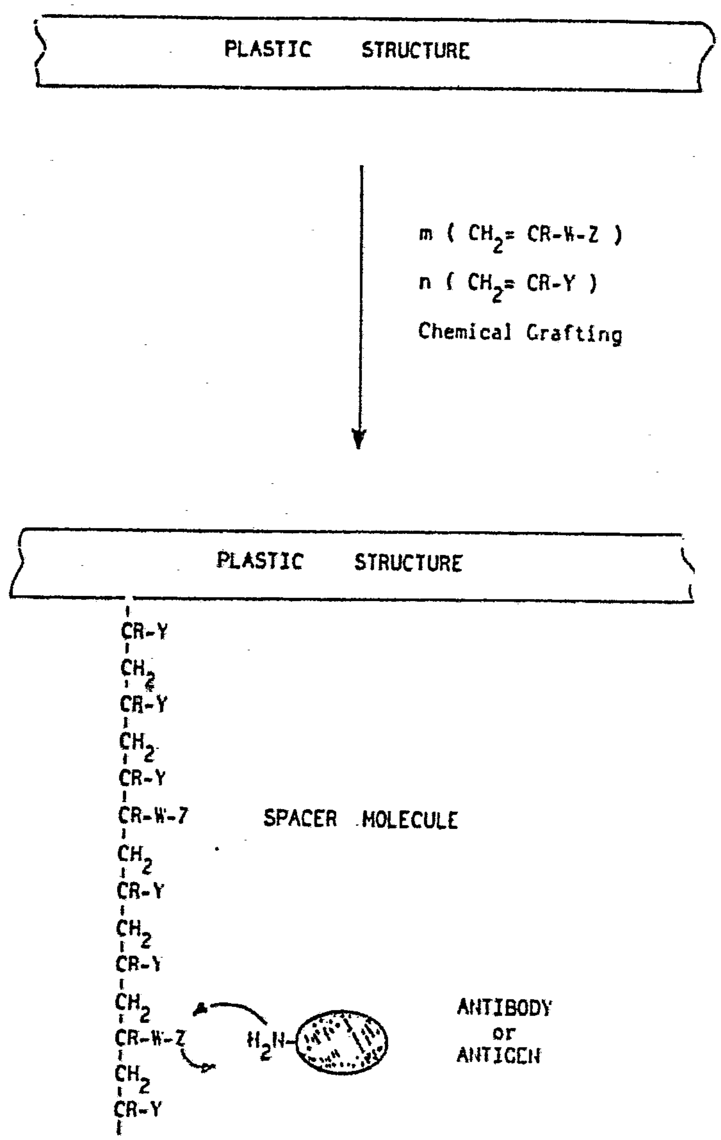
PLASTIC STRUCTURE
m ( CH2= CR-W-Z )
n ( CH2= CR-Y )
Chemical Grafting
PLASTIC STRUCTURE
SPACER MOLECULE
ANTIBODY
or
ANTIGEN

FIGURE 1

This invention relates to a novel instrument and a novel technique for covalent immobilization of molecules having an available amino group. In particular, the invention has application to the detection and quantification of proteins present in low concentrations in body fluids such as serum and, more particularly, to the immobilization of antibodies and antigens to facilitate quantification of antigenic substances by an immunoassay technique.

The determination of the presence or concentration of antigenic substances in serum or other body fluids at low concentrations relies increasingly upon immunoassay techniques, which take advantage of the fact that an antibody binds specifically to its antigen and the reaction obeys the law of mass action, $Ab + Ag \rightleftharpoons (AbAg)$.

Various immunoassay techniques are currently employed and can be classified by their binding characteristics. The most commonly used is competitive binding assays and non-competitive sandwich assays. Regardless of the technique, a labelled antibody or antigen conjugate must be employed for detection. The most widely used labels are radioisotopes, enzymes and fluorescent compounds and the procedure is called radioimmunoassay (RIA), enzyme immunoassay (EIA) and fluorescent immunoassay (FIA), respectively.

Radioimmunoassays utilizing the radioactive isotopes $^{125}I$, $^{14}C$ or $^{3}H$ as labels are still the most widely used assays because of their extremely high sensitivities.

Many of these RIAs can detect as little as $10^{-16}$ moles of antigen or antibody. Despite their high sensitivity, RIAs suffer from a number of disadvantages due to the hazardous nature of the high energy gamma and beta isotopes used in these assays and problems in radioactive waste disposal. In recent years, there have been many attempts to devise new enzyme and fluorescent immunoassay systems which are as sensitive and as rapid as RIAs but which utilize enzymes or fluorescent compounds instead of radioactivity as the means for monitoring the antigen-antibody binding reaction.

Since their first description by Engwall and Perlman in Immunochemistry 8:871 (1971), methods have been developed using enzymes as the labelling compound examples of which are those found in U.S. Patent Numbers 3,654,090, 3,791,932, 3,839,153, 3,850,752, 3,879,262 and those described in the Journal of Immmunological Methods 1:247 (1972) and in Methods of Enzymology 70:419 (1980). Each of the above described methods is based on heterogenous EIA methodology which involves the separation of the reacted enzyme-labelled component from the unreacted enzyme-labelled conjugate by washing. These assays are particularly suitable for the detection and measurement of antigens or antibodies or other substances present in the body fluids which have molecular weights larger than 10,000.

Also of interest are homogenous enzyme-immunoassays described in Biochem. Biophys. Res. Comm. 47:846 (1972) and in U.S. Patent Number 3,817,837. In these assays a hapten is

linked to an enzyme in such a manner that the enzyme activity is altered when the hapten combines with the antibody. No separation steps are required in homogenous EIAs and thus these methods are usually very rapid. These assays are used most widely for measuring therapeutic drug levels, for assaying drugs of abuse and for determination of other antigenic substances with molecular weight lower than 10,000.

Fluorescent immunoassays have sensitivities similar to those of RIAs. As high and variable blanks are quite often encountered, this method appears to be less reliable than RIAs. Several representative RIA methodologies are described in Methods of Enzymology 74:3, 28, 60, 78 and 87 (1981) and in U.S. Patent Number 3,901,654.

Chemiluminescence has also been utilized in immunoassay procedures. Chemiluminescence refers to light produced as a result of chemical change. It involves the transformation of the free energy of a chemical reaction into light energy. A well known chemiluminescent reaction is the oxidation of luminol (5-amino-1, 4-dihydroxyphthalazine) in a basic solution using hydrogen peroxide as an oxidant and in the presence of a catalyst e.g. $Fe^{3+}$, $Cu^{2+}$ or $Co^{2+}$. Some exemplary methods of chemiluminescent immunoassays are described in U.S. Patent Numbers 4,104,029, 4,375,972 and 4,380,580.

Electron spin resonance as a labelling means in immunoassay procedures has been described in U.S. Patent Number 3,850,578. This method requires relatively expensive

instrumentation and free-electron bearing labels which are potentially carcinogenic compounds.

Enzyme immunoassays have found increasing application not only in the fields of clinical analysis but also in microbiology, virology, biochemistry, tissue culturing and immunology. These assays are typically carried out in 96 well (8 x 12 array) plastic microtiter dishes for their convenience of manual handling of large numbers of tests in parallel. The wells (volume 0.3 ml) function as spectrophotometer curvettes with the absorbance of their individual contents being read through the vertical axis by high speed colourimeters having automatic X-Y transport and indexing mechanism. Virtually all commercial microtiter dish EIAs are heterogenous immunoassays and they employ physical adsorption of the primary antibody or antigen to the solid surface of the wells. Although varying with the tertiary structure of the individual antibodies or antigens, relatively low binding densities of about 1 to 200 $ng/cm^2$ are attained through weak hydrophobic interactions of the Van der Waals - London type between the protein and the polymer. Despite the apparent limitations of assaying range and inaccuracy imposed by low binding density and variable desorption of the antibody-antigen complex, adsorptive binding of antibodies and antigens remains widely used in heterogenous competitive RIAs as well as in sandwich type EIAs and FIAs.

Some methods have been recently described to attach an antibody or an antigen through covalent linkage to a solid phase, e.g. to polystyrene beads as reported in the Journal of Immunological Methods 34:315 (1980). In this procedure, polystyrene beads were nitrated and reduced with an alkaline solution of sodium dithionite to generate an aminopolystyrene surface. The beads were activated with glutaraldehyde and used to covalently bind the antibody. These beads, bearing covalently immobilized antibody, were subsequently used in sandwich type immunoassays. Higher surface density of the antibody, shorter incubation times, improved accuracy and range were noted in these assays using covalently bound antibody polystyrene beads. In a similar procedure, described in the Journal of Immunological Methods 57:87 (1983), Schistosoma mansoni antigens were covalently attached to the surface of the wells of 96 well polystyrene microtiter dishes which were used later in enzyme immunoassays for the antibody determination in patients sera. In this work, an aminopolystyrene surface was generated in the wells via the nitration reaction with a mixture of fuming nitric acid in glacial acetic acid and further reduction with alkaline sodium dithionite. The antigenic proteins were coupled to this aminopolystyrene surface with N-ethyl-N'-(3-dimethylaminopropyl)-carbodiimide and suberic acid acting as a short chain coupling molecule to couple a single protein. This report again shows higher sensitivity of the assays carried out with the covalently bound antigens compared to the ones with the physically adsorbed antigens.

The present invention provides a novel method and means for the covalent immobilization of molecules, such as antibodies or antigens, to surfaces of various polymers and provides an instrument that in immunoassay application provides a linear assay relationship over a wide range of concentrations of the substance to be detected and quantified. The invention can also be utilized to immobilize for assay or production purposes any molecule that has an active amino group. For example, it can be used for enzyme immobilization, DNA immobilization, cell immobilization, virus immobilization, bacteria immobilization or in affinity chromatography.

The instrument of the invention takes the form of plastic structures, e.g. vials, curvettes, beads, 96 well microtiter dishes, to which, in immunoassay applications, antibodies can be covalently immobilized in a novel way and used in heterogenous enzyme or fluorescence immunoassays more conveniently than the structures of the prior art where only adhesive protein binding had been utilized and more accurately than existing covalent linkage systems.

In the present invention, for example an antibody (protein) used in the sandwich enzyme or fluorescence immunoassay as the unlabelled antibody, can be covalently immobilized to a polymeric surface by covalently bonding it to a long spacer molecule which is itself covalently attached to the polymer surface. The long spacer moleculer is polymer that has been synthesized and attached covalently to the

polymer surface by chemically or photochemically initiated cografting using two or more appropriate monomers and a free radical initiating system. One of the monomers has the structure $CH_2=CRWZ$ where the group -Z can chemically react with reactive amino acid groups of the protein which thus becomes covalently attached to the spacer molecule. The second monomer provides spacing of the active groups -Z along the spacer molecule and has a structure of $CH_2=CRY$ where the group -Y is a group that can provide the desired hydrophilicity of the long spacer molecule. The cografting of the above monomers can be done in various solvents or mixtures of them at temperatures ranging from zero degrees Celsius in the case of photochemically initiated cografting techniques, and from 40 degrees Celsius in the case of chemically initiated cografting techniques, up to the boiling point temperatures of these solvents in an inert atmosphere. After the grafting is finished, the grafted plastic structures are washed to remove the unreacted monomers, non-grafted polymers, organic solvents and residues of the initiating compounds. The antibody is then covalently immobilized to the activated spacer molecules in an aqueous buffered solution. The plastic structures containing the immobilized antibody are (after neutralizing the residual active groups on the spacer molecules) dried and can be used in the sandwich type or competitive types of enzyme or fluorescence immunoassays.

Accordingly, the invention provides a surface treated polymeric substrate for use in immobilizing molecules containing available amino groups. The substrate has a plurality of a synthesized polymer bonded to it, the polymer comprising a mixture of at least two monomers, one of which contains an active group available for bonding with the amino groups in the molecules to be immobilized whereby multiple active groups are dispersed on the polymers and spaced from the substrate. The invention provides a method for producing this substrate-polymer combination and for its particular use in immunoassay techniques.

With the present invention, therefore, in immunoassay applications, the polyclonal adhesively immobilized antibody presently used in a heterogenous enzyme or fluorescence immunoassay for an antigenic substance is replaced by an antibody covalently immobilized through a long spacer molecule which is itself covalently bound to a polymeric support. The invention is thereby useful for the determination of the concentration or the presence of a wide variety of antigenic substances present in body fluids or other biological materials. The invention will be described herein with reference to immobilizing antigens and antibodies as in immunoassay techniques. Therefore, the term antigen or antigenic substance, as used herein, refers generally to substances to which polyclonal or monoclonal antibodies can be produced against. Among the specific antigens which may be assayed by the process of the present invention may be mentioned human IgG, human IgM, human IgA, mouse IgG and human fibrinogen.

The present invention is also useful for the covalent immobilization of the antigens which may be used in various homogenous competitive radioimmunoassays, enzyme immunoassays and fluorescence immmunoassays. An example of the specific antigens covalently immobilized by the present invention is protein A.

However, the invention can be utilized to bind to a plastic substrate and thereby immobilize for assay purposes, production purposes, etc., molecules that possess an available amino binding group. For example, the invention can be utilized to covalently immobilize polypeptides or polysaccharides, or proteins such as an enzyme, DNA, virus or bacteria, or cells.

These and other features of the invention will be readily apparent from the following description of preferred embodiments thereof, with reference to the accompanying drawings wherein:

Figure 1 is a scheme illustrating the cografting procedure for the formation of the long spacer molecules covalently attached to the polymer surface and the covalent immobilization of an antibody via the activated spacer molecule.

Figure 2 illustrates the results obtained using conventional adhesively bound polyclonal antibodies in three enzyme immunoassays for mouse IgG and human IgG and IgM. Plots for absorbance vs. log of the antigen concentration with the standard error bars ($3\sigma$) are shown.

Figure 3 illustrates the results obtained using the invention, covalently immobilized polyclonal antibodies on long spacers, in three enzyme immunoassays for mouse IgG and human IgG and IgM. Plots for absorbance vs. log concentration with the standard error bars ($3\sigma$) are shown.

As indicated, the invention will be illustrated with reference to the immobilization of antibodies or antigens.

The general method, embodying the invention, for the covalent attachment of antibodies or antigens to the polymeric surface through a spacer molecule is shown in Figure 1 . The main feature of this procedure is not only the use of covalent bonds to, in the end, link the polymer surface and an antibody for its irreversible immobilization but the spacing of the antibody molecule from the polymer surface through a synthesized chain molecule which is described herein as a spacer molecule.

As will be detailed, the spacer molecule is formed by cografting several types of monomers to provide a chain polymer which is covalently bonded to the polymer surface of a plastic substrate. One of the monomers provides active sites "Z" that are dispersed along the spacer molecule and covalently bond to the antibody. The antigen to be measured can then be bonded to the antibody and the tracer or labelled antibody tagged to the antigen for assay purposes.

An antibody molecule linked by a long (up to several hundred Angstroms long) spacer molecule to the polymer surface behaves much more like a dissolved molecule with increased freedom of translation and rotation in contrast to an adsorbed antibody molecule which is practically immobilized on the polymer surface. The higher translational and rotational freedom of the spaced, but immobilized, antibody molecule improves the kinetics of the antigen binding in a typical competitive immunoassay. This effect is even more dramatic in an enzyme sandwich type assay where the presence of the second antibody conjugated with an enzyme further increases steric crowding around the sandwich complex. In this procedure, the incubation time for the antigen binding and for the second antibody-enzyme conjugate can be significantly reduced without a noticeable deterioration of the assaying results.

Importantly, this invention in addition permits excellent control of the density of the covalently immmobilized antibodies or antigens on the polymer surface. The protein surface density of antibodies and antigens obtained in the adhesive binding vary greatly and is a function of the protein structure, its concentration used in the coating solution, the temperature and the pH at which the adsorption procedure is carried out and the surface structure of the plastic to which these antibodies or antigens are adsorbed to. Adhesive binding of the type practiced in the prior art typically produces acceptable densities of the

antibodies on the plastic surfaces although at a cost of usually low utilization yield. The adhesive binding of antigens, particularly those of low molecular weight, is more unreliable and the low surface antigen densities can seriously effect the sensitivity and dynamic range of these assays. As shown in Figure 1, the antibody or antigen molecules containing free amino groups from the lysine residues react with an active group -Z on the spacer molecule creating a covalent bond which permanently immobilizes the protein molecules. In this procedure, therefore, the yield of bound antibody or antigen is significantly increased compared to the prior art's adhesive binding and, moreover, may be easily controlled by a quenching reaction of the reactive -Z groups on the spacer molecules.

Further, the invention permits better control of nonspecific protein binding as compared to the prior art. The nonspecific binding of proteins, e.g. antibody-enzyme conjugates, is a well documented phenomenon and is probably one of the major contributors of errors in heterogenous immunoassays. In standard adhesive enzyme and fluorescence immunoassays, the nonspecific protein binding is suppressed by using a high concentration of an appropriate protein, e.g. bovine serum albumin (BSA) or porcine gelatin, in all assay solutions to saturate the plastic surface and reduce the nonspecific binding of the antibody-enzyme or antibody-fluorophor conjugates. Saturation of the surface with the BSA molecules increases the steric crowding around

the antibody-antigen complex thus decreasing the binding of the antibody-enzyme conjugate which in turn reduces the sensitivity of the immunoassay. However, in the present invention, the antibody or antigen molecules are covalently linked to the polymer surface through the long spacer molecule which effectively spaces them from the surface saturated with BSA or gelatin and therefore introducing negligible steric crowding. Moreover, through use of appropriate monomers in the forming of the spacer molecules, the spacer molecules are of hydrophilic nature which in itself discourages the nonspecific binding.

The covalent binding of antibodies or antigens to polymeric (plastic) structures in the present invention is a two step process. As shown in Figure 1, the spacer molecules are first formed on the surface of the plastic structures by a procedure of the chemically or photochemically initiated cografting of two or more monomers and is followed by the covalent coupling of the antibody or the antigen to the spacer molecules. One of the monomers which is cografted into the spacer molecule and in turn onto the polymer surface is preferably a vinyl monomer with a general structure:

$$CH_2=CR_1-W-Z$$

The group $R_1$ is a hydrogen or a methyl group or an aliphatic or an aromatic group containing up to sixteen carbon atoms and preferably 1 to 6 carbon atoms. The group -Z contains an active ester group and consists essentially of the general structure:

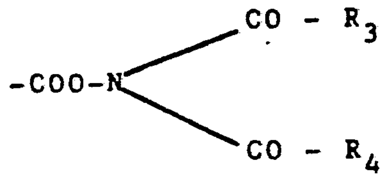

The groups $R_3$ and $R_4$ are hydrogen, or hydrocarbon groups having up to sixteen carbon atoms, and preferably 1 to 6 carbon atoms, in structures that are aliphatic, aromatic or both aliphatic and aromatic and which can contain heteroatoms of oxygen, nitrogen, sulfur, fluorine, bromine and chlorine. Groups $R_3$ and $R_4$ may be bonded together or not bonded together and may when bonded form together an aromatic group. One preferable structure for group Z would contain an active ester group in the following general formula

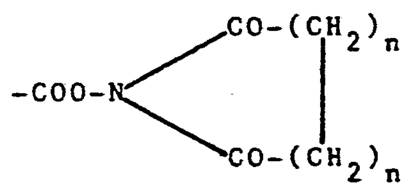

in which n is 1, 2 or 3.

The group -W which links the -Z group to the vinyl group contains up to sixteen carbon atoms, although again preferably 1 to 6 carbon atoms, in aromatic, aliphatic or combinations of aromatic and aliphatic structures and can also contain heteroatoms of oxygen, nitrogen and sulfur. The above-mentioned active esters, which are derivatives of N-diacylhydroxylamines, are known to react easily with the amino groups on lysine residues in proteins forming amide derivatives through which the protein molecule is covalently coupled to the group bearing the active ester. This amide forming reaction has an optimal pH range between pH 7.5 to

8.5 and is known to have a very high ratio of aminolysis/hydrolysis which is about $10^6$, and therefore guarantees high yield of the amide formation even at very low protein concentrations. Another good feature of the active ester groups is that they may be easily quenched by the ammonium ion or other primary amino group containing compounds thus allowing reasonably good control of the protein binding.

The second monomer which is cografted into the spacer molecule has a general formula:

$$CH_2=CR_2-Y$$

where $R_2$ is hydrogen, a methyl group, an aliphatic or an aromatic group containing up to sixteen carbon atoms, and preferably 1 to 6 carbon atoms. The group -Y is an aliphatic and/or aromatic hydrocarbon group having up to sixteen carbon atoms and can contain heteroatoms of oxygen, nitrogen and sulfur. The group provides spacing between the active -Z groups on the spacer molecule and provides desired hydrophilicity in the spacer molecule. Preferably the -Y group is an aliphatic group having 1 to 6 carbon atoms. Typically, when relatively hydrophilic spacers are required, the group Y will contain hydroxyl, amide, carboxyl or similar hydrophilic groups.

A third monomer can also be cografted into the spacer molecule and has a general structure of:

$$CH_2=CR_5-X-R_5C=CH_2$$

Where $R_5$ is a hydrogen, a methyl group, an aliphatic or an aromatic group containing up to sixteen carbon atoms, and

again 1 to 6 carbon atoms is preferable. The -X- group contains up to sixteen carbon atoms in aromatic, aliphatic or combinations of aromatic and aliphatic structures and can contain heteroatoms of oxygen, nitrogen and sulfur. Preferably as well, the -X- group will contain 1 to 6 carbon atoms. These divinyl compounds are added as crosslinking or branching agents and effect the structure of the spacer molecules.

The molar ratio of the monomers $CH_2=CR_1-W-Z$, $CH_2=CR_2-Y$ and $CH_2=CR_5-X-R_5C=CH_2$, present in the grafting procedure does determine the number and the spacing of active groups (binding sites) on the spacer molecule, its hydrophilicity and its crosslinking. It will be apparent that this method will produce in a more spacious environment binding sites that are necessary enabling accurate measurement of high concentrations of material.

The chemically initiated grafting process is initiated with free radical generating compounds like azo compounds, organic peroxides and peroxyesters. All these compounds decompose at certain temperatures generating free radical species which will initiate the polymerization and the grafting reactions. These chemically initiated grafting procedures are done at temperatures ranging from 40 degrees Celsius up to the boiling point temperatures of the solvents used in the process. The most suitable solvents are those which have good solubility and inertness for the monomers and do not dissolve or swell the grafted plastic structure and

have a boiling point above 50 degrees Celsius. Examples of the classes of solvents that may be used are alcohols, ethers, esters and ketones. All grafting procedures must be carried out in an inert atmosphere, typically in nitrogen or argon at atmospheric or increased pressure. The reaction times vary but are typically between two hours and ten hours.

The photochemically initiated grafting process uses UV radiation and photoactive free radical generating compounds like aromatic ketones. These compounds absorb UV radiations of a certain wavelength in high quantum yield and generate free radical species which will initiate the grafting and polymerization reactions. One preferred UV radiation for use in this invention is near-ultraviolet which lies between 320 and 400 nanometers, but preferably around 360 nanometers. This region of the UV spectrum is easily generated with black light fluorescent lamps or high pressure mercury or xenon lamps. Typically, the energy density of the UV irradiations used in this invention range between 25 $Wh/m^2$ and 100 $Wh/m^2$. The photografting procedures are done at temperatures from 0 degrees Celsius up to the boiling temperature of the solvent used in the process.

The same classes of solvents as used in chemically initiated grafting are also used in photografting procedures, most notably alcohols, ethers, esters, and ketones. The preferred photoinitiating compound has been found to be benzophenone with its triplet energy (ET) of 67 Kcal/mol. Inert atmosphere is also typically obtained with nitrogen or

argon at atmospheric pressure. The reaction times vary but typically between 10 minutes and two hours.

The grafted plastic structures are then washed with an organic solvent and deionized water to remove the precipitated copolymer, organic solvent, the unreacted monomers and the decomposition products of the initiating compounds. The grafted plastic structures are typically used immediately for binding of the antibody or the antigen but alternatively can simply be dried in vacuum, sealed into a polyethylene bag and stored refrigerated. When stored under anhydrous conditions, the grafted plastic has been found to remain active for the covalent immobilization of antibodies or antigens for at least six months.

Typical plastic substances used are polystyrene polyvinylchloride, polyethylene, polypropylene, polycarbonate, and polyacrylate.

The covalent coupling of antibodies or antigens to the spacer molecule is carried out in aqueous, non-nucleophilic buffered solutions at pH preferably between 7.5 and 8.5 temperatures ranging from 0 degrees Celsius up to 37 degrees Celsius. The reaction times again range as short as one hour and up to eighteen hours. The protein concentrations in the coupling solutions may be as low as 100 ng/ml but in general for antibodies they are kept around 5 ug/ml. The surface passivation of the antibody or antigen coated plastic surfaces is done with solutions containing bovine serum albumin or egg albumin or non-ionic detergent

like Tween-20$^R$ or mixtures of them. Sometimes it is advantageous to include some ammonium ion in the passivation solution to quench the residual active ester groups on the spacer molecules. The antibody or antigen coated plastic structures may be freeze dried and stored under anhydrous conditions and at sub-zero temperatures for at least twelve months.

With the bonded structure of the invention not only can higher concentrations of matter be captured for measurement, but the optical transparency of the substrate is preserved in higher concentration applications thereby enabling accurate measurement of the bonded matter being assayed.

The enzyme immunoassays for mouse IgG, human IgA, human IgG, human IgM and human fibrinogen exemplified below were performed by using polyclonal goat or rabbit antibodies. All the assays are of the sandwich type using a second antibody-enzyme conjugate with a photometric or fluorometric determination of the product formed by the enzymatic catalysis. The antibody-enzyme conjugates used are either with alkaline phosphatase with p-nitrophenylphosphate as the substrate or horseradish peroxidase with 2,2'-azino-di-(3-ethylbenzthiazoline-6-sulfonate) (ABTS$^R$) as the chromogen. Alkaline phosphatase conjugate was used with the fuorescent substrate 4-methylumbelliferyl phosphate. For the photometric determinations of the enzyme products the Dynatech MR600 96 well plate reader was used. Fluorescent

assays were carried out in a Dynatech Microfluor[R] 96 well plate reader.

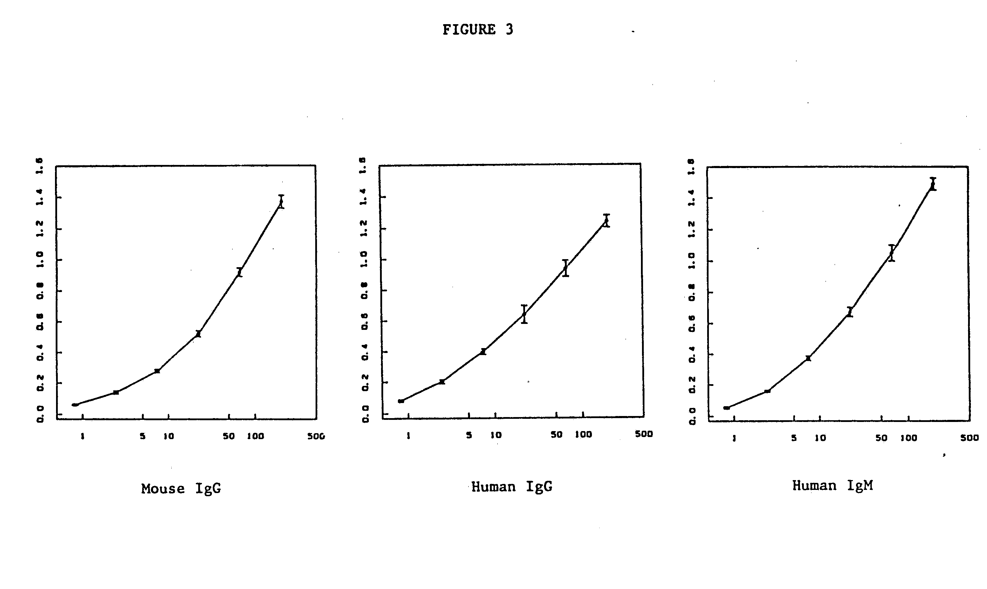

As shown in Figures 2 and 3, a side by side comparison of the enzyme immunoassays for mouse IgG and human IgG and IgM carried out in 96 well plates coated with an antibody by adsorption and in covalently immobilized plates as described provides instant recognition that adsorbed antibody assays degenerate in the high antigen concentration range. The usable assaying range as indicated by overlapping error bars in the plots of Figure 2 is 0.8 ng to 20 ng of the antigen for adsorptively coated plates. In the covalently coated plates as shown in Figure 3, the usable assaying range is extended from 0.8 ng to 200 ng of the antigen. Moreover, the error bars ($3\sigma$) are significantly smaller with the covalently immobilized antibodies due to the higher antibody concentration and their permanent immobilization on the polymeric surface as well as because of lesser steric crowding due to the long spacer molecules immobilizing them and providing them with a much higher freedom for mobility.

It will be apparent that the grafted polymeric substrates can be utilized in any type of assay technique to identify and measure any matter that contains an active amino group that will bind to the active groups sited on the spacer molecule, i.e. the active ester group, or in immunoassay techniques. It matters not what type of labelling is employed and whether enzyme,

fluorescence, radioactive or luminiscence immunoassay is used. In addition, the invention could be used in other applications where immobilization of such a molecule is required, for example DNA alteration.

EXAMPLE 1

1.50 gram of acrylamide (electrophoresis grade purity) and 1.00 gram of N-acryloxysuccinimide was dissolved in 70.0 ml of 2-propanol (containing not more than 0.2% water) with magnetic stirring and at 25 degrees Celsius. Fifty milligrams of 2,2'-azobis-(2-methylpropionitrile) (AZOBIS) was dissolved in 0.50 ml of acetone and this solution was added to the 2-propanol solution which was magnetically stirred for an additional two minutes and filtered through a sintered glass filter. Two polystyrene 96 well microtiter plates (Limbro) were filled with 0.280 ml/well of the filtered 2-propanol solution containing the monomers and the free radical generating AZOBIS. The plates were put into a vacuum oven which was heated to 57 degrees Celsius. The oven was quickly evacuated until the pressure dropped to 100 mbar and then filled with pure argon (99.9%) until the pressure reached 1000 mbar. To further decrease the oxygen concentration in the oven, this process was repeated two more times. The plates were left in the oven for 4.5 hours and at 57 degrees Celsius. A white precipitate appeared in the wells in about one hour. After 4.5 hours, the plates were taken out of the oven and were allowed to

cool for thirty minutes, then they were washed on a 96 well microtiter plate washing apparatus five times with deionized water. The washing procedure required less than three minutes for both plates. The wells of the washed plates were filled with 0.15 ml of a solution of the goat anti mouse IgG antibody (Tago, affinity purified) containing 5 ug/ml of the antibody in a 0.05 M sodium phosphate buffer, pH 8.5. The plates were allowed to stand at 20 degrees Celsius for one hour before being stored at 4 degrees for 18 hours. The plates were then washed three times with deionized water and each well filled with 0.300 ml of sodium phosphate buffer pH 8.5 containing 1% bovine serum albumin and 0.02% of sodium azide. The plates were kept at 20 degrees Celsius for four hours and then at 4 degrees Celsius for another 18 hours. Again these were washed three times with deionized water.

The antigen or sample specimens were diluted in 0.1 M Tris-HCl buffer pH 8.2 containing 1% bovine serum albumin, 0.02% sodium azide and 2 mM calcium chloride and were added to the wells in a volume of 0.150 ml. Each sample or standard was assayed at least in triplicate. Standards used a range of 0.8 ng to 200 ng per well. The plates were incubated at 4 degrees Celsius for 18 hours. Before the addition of the enzyme-antibody conjugate, the plates were washed again three times with deionized water. The goat anti mouse IgG alkaline phosphatase conjugate (Tago affinity purified) was diluted 1:1000 in the 0.1 M Tris-HCl buffer pH 8.2 containing 1% bovine serum albumin 0.02% sodium azide and

2 mM calcium chloride. 0.150 ml of the conjugate solution was delivered to each well with exception of the blank wells. The plates were incubated at 37 degrees Celsius for two hours and at 20 degrees Celsius for one hour. After three wahses with deionized water, the wells were filled with 0.150 ml of 0.5 mM p-nitrophenyl phosphate solution in 1.0 M Tris-HC1 pH 8.2 containing 2 mM magnesium chloride and 0.5 mM zinc chloride. The rates of the absorbance increase at 410 nm in the wells were read on the Dynatech MR 600 automatic plate reader which was interfaced with a Digital Equipment VAX 11/750 computer. The computer acquired the raw kinetic data and performed the statistical and graphical functions as absorbance vs. log of the antigen concentration plot and variance vs. log of the antigen concentration plot.

EXAMPLE 2

1.50 gram of acrylamide (electrophoresis grade purity), 0.70 gram of N-acryloxysuccinimide and 0.030 gram of N,N'-methylyne-bis-acrylamide were dissolved in 70.0 ml of 2-propanol (containing not more than 0.2% water) with magnetic stirring and at 25 degrees Celsius. Fifty milligrams of 2,2'-azabis-(2-methylpropionitrile) (AZOBIS) was dissolved in 0.50 ml of acetone and this solution was added to the 2-propanol solution which was magnetically stirred for an additional two minutes and filtered through a sintered glass filter. Two polystyrene 96 well microtiter plates (Limbro) were filled with 0.280 ml/well of the

filtered 2-propanol solution containing the monomers and the free radical generating AZOBIS. The plates were put into a vacuum oven which was heated to 57 degrees Celsius. The oven was quickly evacuated until the pressure dropped to 100 mbar and then filled with pure argon (99.9%) until the pressure reached 1000 mbar. To further decrease the oxygen concentration in the oven, this process was repeated two more times. The plates were left in the oven for 4.5 hours and at 57 degrees Celsius. A white precipitate appeared in the wells in about one hour. After 4.5 hours, the plates were taken out of the oven and were allowed to cool for thirty minutes, then they were washed on a 96 well microtiter plate washing apparatus five times with deionized water. The washing procedure required less than three minutes for both plates. The wells of the washed plates were filled with 0.150 ml of a solution of the goat anti mouse IgG antibody (Tago, affinity purified) containing 5 ug/ml of the antibody in a 0.05 M sodium phosphate buffer, pH 8.5. The plates were allowed to stand at 20 degrees Celsius for one hour before being stored at 4 degrees Celsius for 18 hours. The plates were then washed three times with deionized water and each well filled with 0.300 ml of sodium phosphate buffer pH 8.5 containing 1% bovine serum albumin and 0.02% of sodium azide. The plates were kept at 20 degrees Celsius for four hours and then at 4 degrees Celsius for another 18 hours. Again these were washed three times with deionized water.

The antigen or serum sample specimens were diluted in 0.1 M Tris-HCl buffer pH 8.0 containing 1% bovine serum albumin, 0.02% sodium azide and 2 mM calcium chloride and were added to the wells in a volume of 0.150 ml. Each sample or standard was assayed at least in triplicate. Standards used a range of 0.8 ng to 200 ng per well. The plates were incubated at 4 degrees Celsius for 18 hours. Before the addition of the enzyme-antibody conjugate, the plates were washed again three times with deionized water.

The goat anti mouse IgG horseradish peroxidase conjugate (Tago, affinity purified) was diluted 1:1000 in the 0.05 M sodium phosphate buffer pH 8.5 containing 1% bovine serum albumin, 0.01% methiolate and 1% sodium chloride. 0.150 ml of the conjugate solution was delivered to each well with exception of the blank well. The plates were incubated at 20 degrees Celsius for one hour. After three washes with deionized water, the wells were filled with 0.150 ml of 0.1% 2.2'-azino-di-(3-ethylbenzthiazoline-6-sulfonate) (ABTS) in a citrate/phosphate buffer pH 5.0 containing 0.003% hydrogen peroxide. The absorbance in the wells were read at 410 nm on the Dynatech MR 600 automatic plate reader interfaced with a Digital Equipment VAX 11/750 computer performing the statistical and graphical functions as described in Example 1.

EXAMPLE 3

A solution of 21.5 gram of acrylamide (electrophoresis grade purity) and 10.0 gram of N-acryloxysuccinimide was prepared in 1000 ml of 2-propanol (containing not more than 0.2% water) with magnetic stirring and at 25 degrees Celsius. Another solution containing 0.72 gram of AZOBIS was prepared in 5.0 ml of acetone and was added under stirring to the 2-propanol solution. This solution filtered through a sintered glass filter and was delivered to the wells of thirty-six 96 well microtiter plates (Limbro) in a 0.280 ml/well dose.

The plates were put into a vacuum oven which was heated to 57 degrees Celsius. The oven was quickly evacuated until the pressure dropped to 100 mbar and then filled with pure argon (99.9%) until the pressure reached 1000 mbar. To further decrease the oxygen concentration in the oven this process was repeated two more times. The plates were left in the oven for 4.5 hours at 57 degrees Celsius. A white precipitate appeared in the wells in about one hour. After 4.5 hours, the plates were taken out of the oven and were allowed to cool for thirty minutes, then they were washed on a 96 well microtiter plate washing apparatus five times with deionized water.

The wells of the washed plates were filled with 0.150 ml of a solution of goat anti human IgM antibody (Tago, affinity purified) containing 5 ug/ml of the antibody in a 0.05 M sodium phosphate buffer, pH 8.5. The plates were

allowed to stand at 20 degrees Celsius for one hour before being stored at 4 degrees Celsius for 18 hours. The plates were then washed three times with deionized water and each well filled with 0.300 ml of sodium phosphate buffer pH 8.5 containing 1% bovine serum albumin and 0.02% of sodium azide. They were kept at 20 degrees Celsius for four hours and then at 4 degrees Celsius for another 18 hours. They were washed three times with deionized water and the residual water was shaken out of the wells and finally sealed in polyethylene bags and stored at -18 degrees Celsius. These plates with covalently immobilized goat anti human IgM have not shown any deterioration in enzyme immunoassays for human IgM for twelve months since stored at -18 degrees Celsius.

EXAMPLE 4

A solution of 19.3 gram of acrylamide (electrophoresis grade purity) and 9.0 gram of N-acryloxysuccinimide was prepared in 900 ml of 2-propanol (containing not more than 0.2% water) with magnetic stirring and at 25 degrees Celsius. Another solution containing 0.64 gram of AZOBIS was prepared in 15.0 ml of acetone and was added under stirring to the 2-propanol solution. This solution was filtered through a sintered glass filter and was delivered to the well of thirty-six 96 well microtiter plates (Limbro) with 0.280 ml/well.

The plates were put into a vacuum oven which was heated to 57 degrees Celsius. The oven was quickly evacuted

until the pressure dropped to 100 mbar and then filled with pure argon (99.9%) until the pressure reached 1000 mbar. To further decrease the oxygen concentration in the oven, this process was repeated two more times. The plates were left in the oven for 4.5 hours and at 57 degrees Celsius. A white precipitate appeared in the wells in about one hour. After 4.5 hours, the plates were taken out of the oven and were allowed to cool for thirty minutes. The plates were then washed on a 96 well microtiter plate washing apparatus five times with deionized water. The plates were dried in vacuum at 0.1 mbar and at 25 degrees Celsius for twenty-four hours and were immediately sealed in polyethylene bags containing DRIERITE$^R$ to adsorb the residual moisture in the bag which could hydrolyze the active ester groups on the spacer molecules. These plates have shown no loss of activity for antibody or antigen binding for three months when stored at 20 degrees Celsius and for six months when stored at 4 degrees Celsius.

## EXAMPLE 5

Two 96 well plates (Dynatech, Microfluor "B"$^R$, black polystyrene for fluorescent immunoassays) were grafted with N-acryloxysuccinimide and acrylamide and coated with goat anti mouse IgG (Tago) exactly as described in Example 2.

The antigen or sample specimens were diluted in 0.1 M Tris-HCl buffer pH 8.2 containing 1% bovine serum albumin, 0.02% sodium azide and 2mM calcium chloride and were added in

a volume of 0.150 ml per well. Each sample or standard was assayed at least in triplicate. Standards used a range of 33 picograms to 9.0 nanograms (9000 picograms). The plates were incubated at 4 degrees Celsius for 18 hours and then they were washed three times with deionized water.

Goat anti mouse IgG alkaline phosphatase conjugate (Tago, affinity purified) was diluted 1:1000 in 0.1 M Tris-HCl pH 8.2 containing 1% bovine serum albumin, 0.02% sodium azide and 2 mM calcium chloride. 0.150 ml of the conjugate solution was delivered to each well except into the blank well. The plates were incubated at 37 degrees Celsius for two hours and at 20 degrees Celsius for one hour. After three washes with deionized water, the wells were filled with 0.200 ml of 0.5 mM 4-methylumbelliferyl phosphate in 1.0 M Tris HCl pH 8.2 containing 2 mM magnesium chloride and 0.5 mM zinc chloride. The rates of fluorescence increase were measured on a Dynatech MicroFLOUR TM reader. The excitation wavelength was at 350 nm and the emission was measured at 460 nm. The raw kinetic data were supplied to the Digital Equipment VAX 11/750 computer which performed the statistical and graphical functions as fluorescence (arbitrary units) vs. log of the antigen concentration and variance vs. log of the antigen concentration plot.

## EXAMPLE 6

Two 96 well microtiter polystyrene plates (Limbro) were grafted with N-acryloxysuccinimide and acrylamide

exactly as described in Example 2. Procedures for coating the plate with the first antibody (goat anti mouse IgG) and subsequent deactivation with 1% BSA were the same as described for Example 1. However, the alkaline phosphatase labelled second antibody was added first (1:500 dilution) in 75 ul of buffer to each well and was followed immediately by the sample or standard in the same volume of buffer. The plate was then incubated at 37 degrees Celsius for two hours. The rest of the procedures for substrate addition and the reading of absorption at 410 were identical to that of the regular assay as described in Example 1.

## EXAMPLE 7

Two 96 well microtiter polystyrene plates (Limbro) were grafted with N-acryloxysuccinimide and acrylamide exactly as described in Example 2.

Plates are coated with human fibrinogen dissolved in 0.05 M sodium phosphate buffer pH 8.5 at 4 degrees Celsius overnight. Ten wells are used for each concentration of fibrinogen tested. Amount of fibrinogen used per well are 2000, 500, 100, 50, 10 and 1 ng in a volume of 0.15 ml. After washing, the plate is deactivated with a 1% BSA solution in 0.05 M phosphated buffer pH 8.5 at room temperature for 4 hours.

With the excess BSA removed after washing, horseradish peroxidase labelled goat anti-human fibrinogen antibody at 1:500 dilution in 0.05 M phosphate buffer

containing 1% NaCl, 1% BSA and 0.01% methiolate is added to the plate. Each well receives 0.15 ml of the antibody. Plate is incubated at room temperature with the enzyme for one and a half hours. After washing, 0.15 ml of 1% ABTS in a citrate/phosphate buffer pH 5.0 with 0.03% hydrogen peroxide is added to each well. Plate is read 5 minutes after addition of substrate.

EXAMPLE 8

1.50 gram of acrylamide (electrophoresis grade purity) and .700 gram of N-acryloxysuccinimide was dissolved in 70.0 ml of 2-propanol (containing not more than 0.2% water) with magnetic stirring and at 25 degrees Celsius. 50.0 milligram of 2,2'-azobis-(2-methylpropionitrile) (AZOBIS) was dissolved in 0.50 ml of acetone and this solution was added to the 2-propanol solution which was magnetically stirred for an additional two minutes and filtered through a sintered glass filter. Two polyvinyl 96 well microtiter plates (Dynatech) were filled with 0.20 ml/well of the filtered 2-propanol solution containing the monomers and the free radical generating AZOBIS. The plates were put into a vacuum oven which was heated to 57 degrees Celsius. The oven was quickly evacuated until the pressure dropped to 100 mbar and then filled with pure argon (99.9%) until the pressure reached 1000 mbar. To further decrease the oxygen concentration in the oven, this process was repeated two more times. The plates were left in the oven for 4.5 hours and at

57 decrees Celsius. A white precipitate appeared in the wells in about one hour. After 4.5 hours, the plates were taken out of the oven and were allowed to cool for thirty minutes, then they were washed on a 96 well microtiter plate washing apparatus five times with deionized water. The washing procedure required less than three minutes for both plates. The wells of the washed plates were filled with 0.150 ml of a solution of the goat anti mouse IgG antibody (Tago, affinity purified) containing 5 ug/ml of the antibody in a 0.05 M sodium phosphate buffer, pH 8.5. The plates were allowed to stand at 20 degrees Celsius for one hour before being stored at 4 degrees Celsius for 18 hours. The plates were then washed three times with deionized water and each well filled with 0.20 ml of sodium phosphate buffer pH 8.5 containing 1% bovine serum albumin and 0.02% of sodium azide. The plates were kept at 20 degrees Celsius for four hours and then at 4 degrees Celsius for another 18 hours. Again, these were washed three times with deionized water.

The antigen or sample specimens were diluted in 0.1 M Tris-HCl buffer pH 8.2 containing 1% bovine serum albumin, 0.02% sodium azide and 2 mM calcium chloride and were added to the wells in a volume of 0.150 ml. Each sample or standard was assayed at least in triplicate. Standards used a range of 0.8 ng to 200 ng per well. The plates were incubated at 4 degrees Celsius for 18 hours. Before the addition of the enzyme-antibody conjugate, the plates were washed again three times with deionized water. The goat anti

mouse IgG alkaline phosphatase conjugate (Tago, affinity purified) was diluted 1:1000 in the 0.1 M Tris-HCL buffer pH 8.2 containing 1% bovine serum albumin, 0.02% sodium azide and 2 mM calcium chloride. 0.150 ml of the conjugate solution was delivered to each well with exception of the blank well. The plates were incubated at 37 degrees Celsius for two hours and at 20 degrees Celsius for one hour.

After three washes with deionized water, the wells were filled with 0.150 ml of 0.5 mM p-nitrophenyl phosphate solution in 1.0 M Tris-HCL pH 8.2 containing 2 nM magnesium chloride and 0.5 mM zinc chloride. The rates of the absorbance increase at 410 nm in the wells were read on the Dynatech MR 600 automatic plate reader which was interfaced with a Digital Equipment VAX 11/750 computer. The computer acquired the raw kinetic data and performed the statistical and graphical functions as absorbance vs. log of the antigen concentration plot and variance vs. log of the antigen concentration plot.

EXAMPLE 9

1.50 gram of acrylamide (electrophoresis grade purity) and 0.70 gram of N-acryloxysuccinimide was dissolved in 70.0 ml of 2-propanol (containing not more than 0.2% water) with magnetic stirring and at 25 degrees Celsius. 50.0 ul of a-cumyl-peroxyneodecanoate was dissolved in 0.50 ml of acetone and this solution was added to the 2-propanol solution which was magnetically stirred for an additional two

minutes and filtered through a sintered glass filter. Two polystyrene 96 well microtiter plates (Limbro) were filled with 0.280 ml/well of the filtered 2-propanol solution containing the monomers and the free radical generating a-cumyl-peroxyneodecanoate. The plates were put into a vacuum oven which was heated to 57 degrees Celsius. The oven was quickly evacuated until the pressure dropped to 100 mbar and then filled with pure argon (99.9%) until the pressure reached 1000 mbar. To further decrease the oxygen concentration in the oven, this process was repeated two more times. The plates were left in the oven for 4.5 hours and at 57 degrees Celsius. A white precipitate appeared in the wells in about one hour. After 4.5 hours, the plates were taken out of the oven and were allowed to cool for thirty minutes, then they were washed on a 96 well microtiter plate washing apparatus five times with deionized water. The washing procedure required less than three minutes for both plates. The wells of the washed plates were filled with 0.150 ml of a solution of the goat anti mouse IgG antibody (Tago, affinity purified) containing 5 ug/ml of the antibody in a 0.05 M sodium phosphate buffer, pH 8.5. The plates were allowed to stand at 20 degrees Celsius for one hour before being stored at 4 degrees Celsius for 18 hours. The plates were then washed three times with deionized water and each well filled with 0.300 ml of sodiumphosphate buffer pH 8.5 containing 1% bovine serum albumin and 0.02% of sodium azide. The plates were kept at 20 degrees Celsius for four hours and

then at 4 degrees Celsius for another 18 hours. Again these were washed three times with deionized water.

The antigen or sample specimens were diluted in 0.1 M Tris-HCl buffer pH 8.2 containing 1% bovine serum albumin, 0.02 % sodium azide and 2 mM calcium chloride and were added to the wells in a volume of 0.150 ml. Each sample or standard was assayed at least in triplicate. Standards used a range of 0.8 ng to 200 ng per well. The plates were incubated at 4 degrees Celsius for 18 hours. Before the addition of the enzyme-antibody conjugate, the plates were washed again three times with deionized water. The goat anti mouse IgG alkaline phosphatase conjugate (Tago, affinity purified) was diluted 1:1000 in the 0.1 M Tris-HCl buffer pH 8.2 containing 1% bovine serum albumin, 0.02% sodium azide and 2 mM calcium chloride. 0.150 ml of the conjugate solution was delivered to each well with exception of the blank well. The plates were incubated at 37 degrees Celsius for two hours and at 20 degrees Celsius for one hour. After three washes with deionized water, the wells were filled with 0.150 ml of 0.5 mM p-nitrophenyl phosphate solution in 1.0 M Tris HCl pH 8.2 containing 2 mM magnesium chloride and 0.5 mM zinc chloride. The rates of the absorbance increase at 410 nm in the wells were read on the Dynatech MR 600 automatic plate reader which was interfaced with a Digital Equipment VAX 11/700 computer. The computer acquired the raw kinetic data and performed the statistical and graphical functions as absorbance vs. log of the antigen concentration plot and variance vs. log of the antigen concentration plot.

EXAMPLE 10

0.38 gram of acrylamid (electrophoresis grade purity) and 0.25 gram of N-acryloxysuccinimide were dissolved in 70.0 ml of 1-propanol (containing not more than 0.2% water) with magnetic stirring at 25 degrees Celsius. 1.25 grams of benzophenone were added to the 1-propanol solution which was stirred for an additional 10 minutes and filtered through a sintered glass funnel. Two polystyrene 96 well microtiter plates (Limbro) were filled with 0.280 ml/well of the filtered 1-propanol solution containing the monomers and the photoinitiating compound benzophenone generating the free radical species via UV light activation. The plates were put into a flat bottom glass chamber (L 12" x W 12" x H 2") and flushed with a stream of pure argon for 15 minutes to remove oxygen. The chamber was positioned one inch vertically above an array of nine black light fluorescent lamps spaced at 1/4 inch longitudinally from each other. This light source (Sylvania F15T8/BLB fluorescent lamp, 18" x 7/8") delivered 40 $Wh/m^2$ of UV radiation energy at 360 nanometers at the bottom glass plate of the grafting chamber. The lamps and the chamber were cooled by forced air and the measured temperature in the chamber was 25 degrees Celsius.

A white precipitate appeared in the wells ten minutes after the UV lamps were switched on. After 1 hour, the UV lamps were turned off, the plates washed on a 96 well microtiter plate washing apparatus two times with 2-propanol to remove the water insoluble photoinitiator, benzophenone.

Then the plates were washed five times with deionized water on the same washing apparatus. The washing procedure required less than five minutes for both plates. Procedures for coating the plates with first antibody (Tago goat anti-mouse IgG) and subsequent deactivation with BSA, addition of the standards and sample (mouse IgG), addition of horseradish peroxidase labelled second antibody, substrate addition and the measurement of the absorption at 410 nanometers were identical to that of the assay as described in Example 2.

EXAMPLE 11

0.38 gram of acrylamide (electrophoresis grade purity), 0.25 gm of N-acryloxysuccinimide and 1.25 grams of benezophenone were dissolved in 70 ml of 1-propanol (containing not more than 0.2% water) with magnetic stirring at 25 degrees Celsius. The solution was filtered through a sintered glass filter and two polyvinyl chloride 96 well plates (Dynatech) were filled with the 1-propanol solution (0.20 ml/well). The plates were put into a flat bottom glass chamber (L 12" x W 12" x H2") and flushed with a stream of pure argon for 15 minutes to remove oxygen. The chamber was positioned one inch vertically above an array of nine black light fluorescent lamps spaced at 1/4 inch longitudinally from each other. This light source (Sylvania F15T8/BLB fluorescent lamp, 18" x 7/8") delivered 40 $Wh/m^2$ of UV radiation energy at 360 nanometers at the bottom glass plate of the grafting chamber.

The photoinitiated grafting was done for 30 minutes and then the plates were washed on a 96 well microtiter plate washing apparatus two times with 2-propanol to remove benzophenone followed with five washes with deionized water on the same washing apparatus. The washing procedure required less than five minutes for both plates. Procedures for coating the plates with first antibody (Tago goat anti mouse IgG) and subsequent deactivation with BSA, addition of the standards and sample (mouse IgG), addition of horseradish peroxidase labelled second antibody, substrate addition and the measurement of the absorption at 410 nanometers were identical to that of the assay as described in Example 2.

EXAMPLE 12

1.15 grams of acrylamide (electrophoresis grade purity), 0.75 gram of N-acryloxysuccinimide and 1.250 grams of benzophenone were dissolved in 210 ml of 1-propanol (containing no more than 0.2% water) with magnetic stirring at 25 degrees Celsius. The solution was filtered through a sintered glass filter and six polystyrene 96 well microtiter plates (Limbro) were filled (0.28 ml/well) with this solution containing the monomers and the photoinitiating compound. The plates were put into the photografting apparatus. The grafting and washing procedures for the plates were identical to that described in Example 11. The plates were dried in vacuum at 0.1 mbar at 25 degrees Celsius for twenty-four hours and were immediately sealed in polyethylene bags containing DRIERITE$^R$ to absorb the residual moisture in the bag which could hydrolyse the active ester groups on the

spacer molecules. The activated plates have shown no loss of activity for antibody or antigen binding for three months when stored at 20 degrees Celsius and for six months when stored 4 degrees Celsius.

EXAMPLE 13

0.75 gram of acrylamide (electrophoresis grade purity), 0.50 gram of N-acryloxysuccinimide and 1.25 grams of benzophenone were dissolved in 50 ml acetone with magnetic stirring at 0 degrees Celsius and 20 ml of ice cold deionized water containing 0.1 ml glacial acetic acid was added slowly with stirring. The resulting homogenous solution was filtered quickly through a precooled sintered glass funnel to minimize the hydrolysis of the active ester, N-acryloxysuccinimide.

The filling and photografting procedures of two polystyrene 96 well microtiter plates (Limbro) were done in a cold room at 4 degrees Celsius for 30 minutes. The plates were then washed on a 96 well microtiter plate washing appartus for two times with 2-propanol and five times with ice cold deionized water. Procedures for coating the plates with the first antibody (Tago, goat anti mouse IgG), subsequent deactivation with 1% BSA, addition of the standards and sample (mouse IgG), addition of horseradish peroxidase labelled second antibody, addition of the substrate and the measurement of absorption at 410 nanometers were identical to that of the assay as described in Example 2.

I CLAIM:

1. A surface treated polymeric substrate for use in immobilizing molecules containing available amino groups, the substrate having at least one polymer bonded thereto, same polymer comprising a mixture of at least two monomers, one of which contaims an active group available for bonding with amino groups in said molecules to be immobilized whereby multiple active groups are dispersed on said polymer and spaced from said substrate.

2. A surface treated polymeric substrate as claimed in claim 1, further comprising a plurality of molecules having an available amino group covalently bonded by that amino group each to one of said active groups dispersed on said polymer.

3. A surface treated polymeric substrate for use in immobilizing molecules containing available amino groups, the substrate having at least one polymer bonded thereto said polymer comprising a mixture of:

a monomer of the general formula

$$CH_2=CR_1-W-Z \qquad I$$

wherein

$R_1$ is hydrogen, a methyl group or an aliphatic group or aromatic group having up to sixteen carbon atoms

Z is a group having the general formula

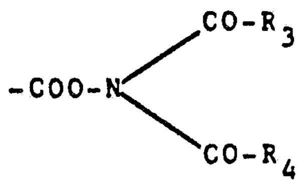

II

wherein

$R_3$ and $R_4$ are unbonded or bonded together and are hydrogen, aliphatic, aromatic or aliphatic and aromatic groups having up to sixteen carbon atoms, or aliphatic, aromatic or aliphatic and aromatic groups having up to sixteen carbon atoms and having heteroatoms of oxygen, nitrogen, sulfur, fluorine, bromine and/or chlorine, and when bonded may form a single aromatic group, and W is an aliphatic, aromatic or aliphatic and aromatic group having up to sixteen carbon atoms, or an aliphatic, aromatic, or aliphatic and aromatic group having up to sixteen atoms,

and a monomer of the general formula

$$CH_2=CR_2-Y \qquad III$$

wherein

$R_2$ is hydrogen, a methyl group or an aliphatic or an aromatic group having up to sixteen carbon atoms, and Y is an aliphatic, an aromatic or an aliphatic and aromatic group having up to sixteen carbon atoms, or an aliphatic, aromatic or aliphatic and aromatic group having up to sixteen carbon atoms and containing heteroatoms of oxygen, nitrogen and sulfur.

4. A surface treated polymeric substrate as claimed in claim 3 in which group Z is of the general formula:

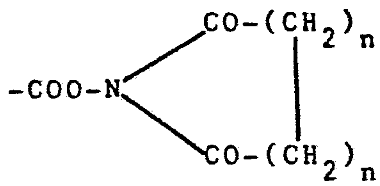

wherein

n is 1, 2 or 3, and

group Y is an aliphatic group having 1 to 6 carbon atoms and groups $R_1$, $R_2$, X and W contain 1 to 6 carbon atoms.

5. A surface treated polymeric substrate as claimed in claims 3 or 4 in which Y contains a hydrophilic group.

6. A surface treated polymeric substrate as claimed in claims 3 or 4 in which Y contains a hydrophilic group comprising one of the group consisting of hydroxyl, amide and carboxyl.

7. A surface treated polymeric substrate as claimed in claim 3 in which said polymer further contains a monomer of the general formula

$$CH_2=CR_5-X-RC_5=CH_2 \qquad IV$$

wherein

R is hydrogen, a methyl group, or an aliphatic or aromatic group having up to sixteen carbon atoms

X is an aliphatic, an aromatic or an aliphatic and aromatic group having up to sixteen carbon atoms or an aliphatic and aromatic group having up to sixteen carbon atoms and having heteroatoms of oxygen, nitrogen or sulfur.

8. A surface treated polymeric substrate as claimed in claim 4 in which said polymer further contains a monomer of the general formula

$$CH_2=CR_5-X-R_5C=CH_2 \qquad V$$

wherein

$R_5$ is hydrogen, a methyl group, or an aliphatic or aromatic group having one to six carbon atoms.

X is an aliphatic, an aromatic or an aliphatic and aromatic group having one to six carbon atoms, or an aliphatic and aromatic group having one to six carbon atoms and having heteroatoms of oxygen, nitrogen or sulfur.

9. A surface treated polymeric substrate as claimed in claims 3, 4 or 7, further comprising a plurality of molecules having an active amino group covalently bonded by that amino group each to one of said Z groups dispersed on said polymer.

10. A surface treated polymeric substrate as claimed in claims 3, 4 or 7 further comprising a plurality of antibody or antigen molecules each covalently bonded by their amino groups to one of the said Z groups dispersed on said polymer.

11. A surface treated polymeric substrate as claimed in claims 3, 4 or 7 further comprising a plurality of one of the group consisting of a polypeptide and a polysaccharide each covalently bonded by their amino groups to one of said Z groups dispersed on said polymer.

12. A surface treated polymeric substrate as claimed in claims 3, 4 or 7 in which said polymeric substrate comprises one of the group consisting of a polystyrene, a polyvinylchloride, a polyethylene, a polypropylene, a polycarbonate and a polyacrylate.

13. A process for the preparation of a surface treated polymeric substrate as claimed in claim 1 wherein said monomers are polymerized and grafted to said substrate in an inert atmosphere and in the presence of a solvent inert to the said monomers by treatment with free radical generating compounds that are decomposed under one of chemical and photochemical influence to produce free radical compounds to initiate the grafting and polymerization reactions.

14. A process for the preparation of a surface treated polymeric substrate as claimed in claim 3 in which monomers of the general formula

$$CH_2=CR_1-W-Z \quad \text{I}$$

wherein $R_1$, W and Z are as defined in claim 3 and monomers of the general formula

$$CH_2=CR_2-Y \quad \text{II}$$

wherein $R_2$ and Y are as defined in claim 3,

(i) are dissolved in a solution of solvent inert to the said monomers and treated, in an inert atmosphere in the presence of said polymeric substrate, and at a

temperature in the range of 40 degrees Celsius to the boilng point of the solvent, with free radical generating compounds that decompose at temperatures within the said range to produce free radical compounds,

(ii) washing the said polymeric substrate with deionized water,

whereby a plurality of said polymers are formed and bonded to said polymeric substrate.

15. A process for the preparation of a surface treated polymeric substrate as claimed in claim 3 in which monomers of the general formula

$$CH_2=CR_1-W-Z \qquad I$$

wherein $R_1$, W and Z are as defined in claim 3 and monomers of the general formula

$$CH_2=CR_2-Y \qquad II$$

wherein $R_2$ and Y are as defined in claim 3,

(i) are dissolved in a solution of solvent inert to the said monomers, said solvent containing photoactive free radical generating compounds, and treated with radiation in an inert atmosphere in the presence of said polymeric substrate and at a temperature in the range of 0 degrees Celsius to the boiling point of

the solvent, said radiation promoting the generation of free radical compounds from said free radical generating compounds,

(ii) washing the said polymeric substrate with deionized water,

whereby a plurality of said polymers are formed and bonded to said polymeric substrate.

16. A process as claimed in claim 14 or 15 which comprises the further step of

(iii) drying the said polymeric substrate.

17. A process as claimed in claim 14 in which the solvent comprises one of the group consisting of an alcohol, ether, ester and ketone, and the free radical generating compounds comprise one or more of the group consisting of azo compounds, organic peroxides and peroxyesters and the inert atmosphere comprises one of the group consisting of nitrogen and argon.

18. A process as claimed in claim 15 in which the solvent comprises one of the group consisting of an alcohol, ether, ester and ketone, and the free radical generating compounds comprises an aromatic ketone, and the inert atmosphere comprises one of the group consisting of nitrogen and argon.

19. A process as claimed in claim 15 in which the free radical generating compound comprises benzophenone.

20. The process of claim 14 or 17 in which the reaction time for step (i) is two to ten hours.

21. The process of claim 15, 18 or 19 in which the reaction time for step (i) is ten minutes to two hours.

22. A process as claimed in claim 14 which comprises the further step of

(iii) treating said surface treated polymeric substrate with a plurality of molecules each having an available amino group, in an aqueous, non-nucleophilic buffered solution at a pH in the range of 7.5 to 8.5 at a temperature in the range of 0 degrees Celsius to 37 degrees Celsius and in the presence of a hydrolyzing agent, whereby said molecules are covalently bonded by their amino group each to one of said active Z groups dispersed on said polymer.

23. A process as claimed in claim 15 which comprises the further step of

iii) treating said surface treated polymeric substrate with a plurality of molecules each having an available amino group, in an aqueous, non-nucleophilic buffered solution at a pH in the range of 7.5 to 8.5 at a temperature in the range of 0 degrees Celsius to 37 degrees Celsius and in the presence of a hydrolyzing agent, whereby said molecules are covalently bonded by their amino group each to one of said active Z groups dispersed on said polymer.

24. A process as claimed in claim 22 or 23 in which the hydrolyzing agent comprises one of the group consisting of bovine serum albumin, egg albumin, non-ionic detergent and mixtures thereof.

25. A process as claimed in claim 22 or 23 in which said molecule having an available amino group comprises an antibody or an antigen.

26. In an immunoassay technique, the step of utilizing a surface treated polymeric substrate as claimed in claims 1, 3 or 4 to immobilize antigens or antibodies by bonding their amino groups to the active groups of the polymer whereby the antigens or antibodies are immobilized in spaced relationship to said substrate.

27. In a technique for immobilizing molecules having available amino groups, the step of utilizing a surface treated polymeric substrate as claimed in claims 1, 3 or 4 to immobilize said molecules by bonding their amino groups to the active groups of the polymer whereby the molecules are immobilized in spaced relationship to said substrate.

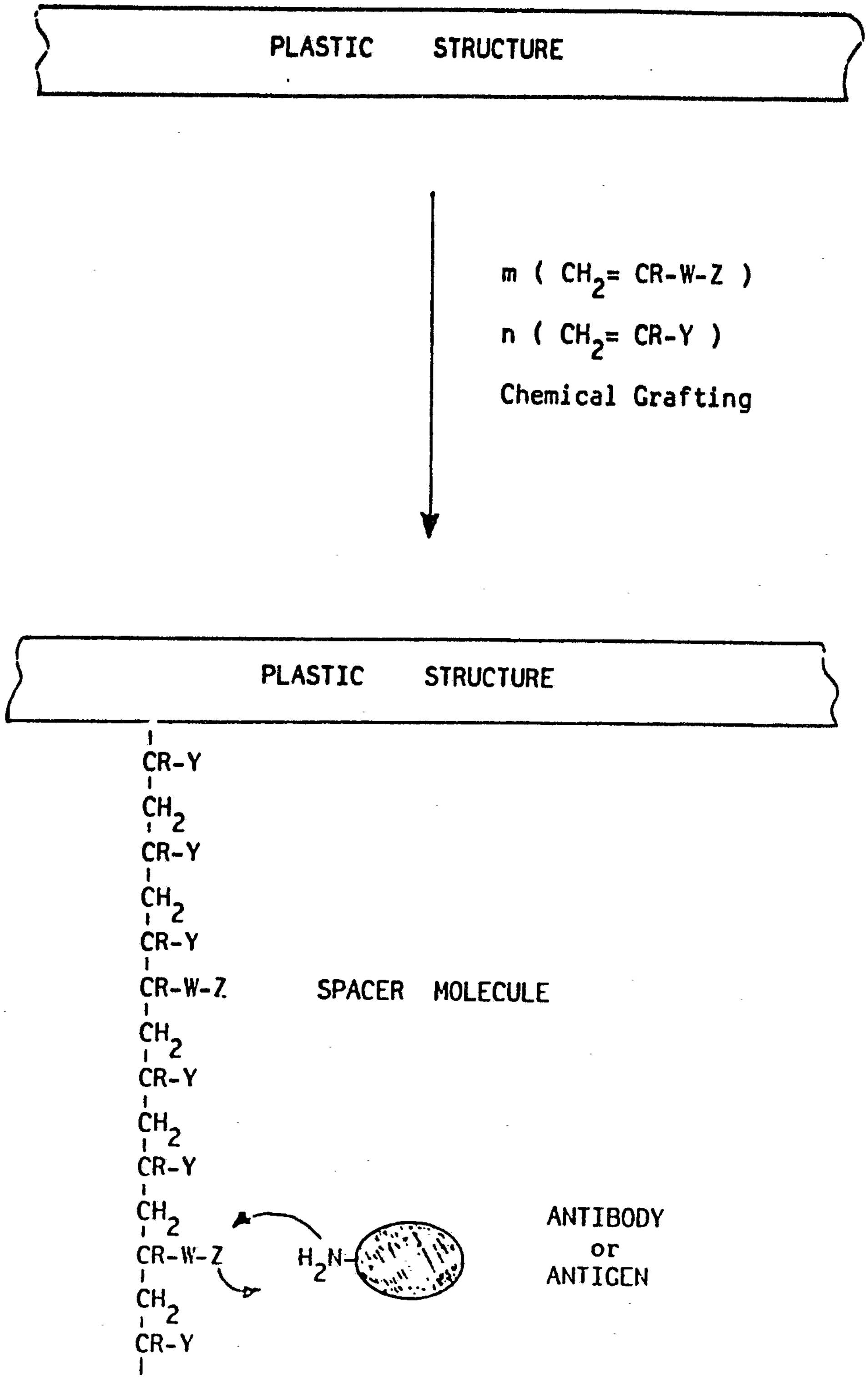
PLASTIC STRUCTURE
m ( $CH_2$= CR-W-Z )
n ( $CH_2$= CR-Y )
Chemical Grafting
PLASTIC STRUCTURE
SPACER MOLECULE
ANTIBODY
or
ANTIGEN

FIGURE 1

2/3

0164889

FIGURE 2

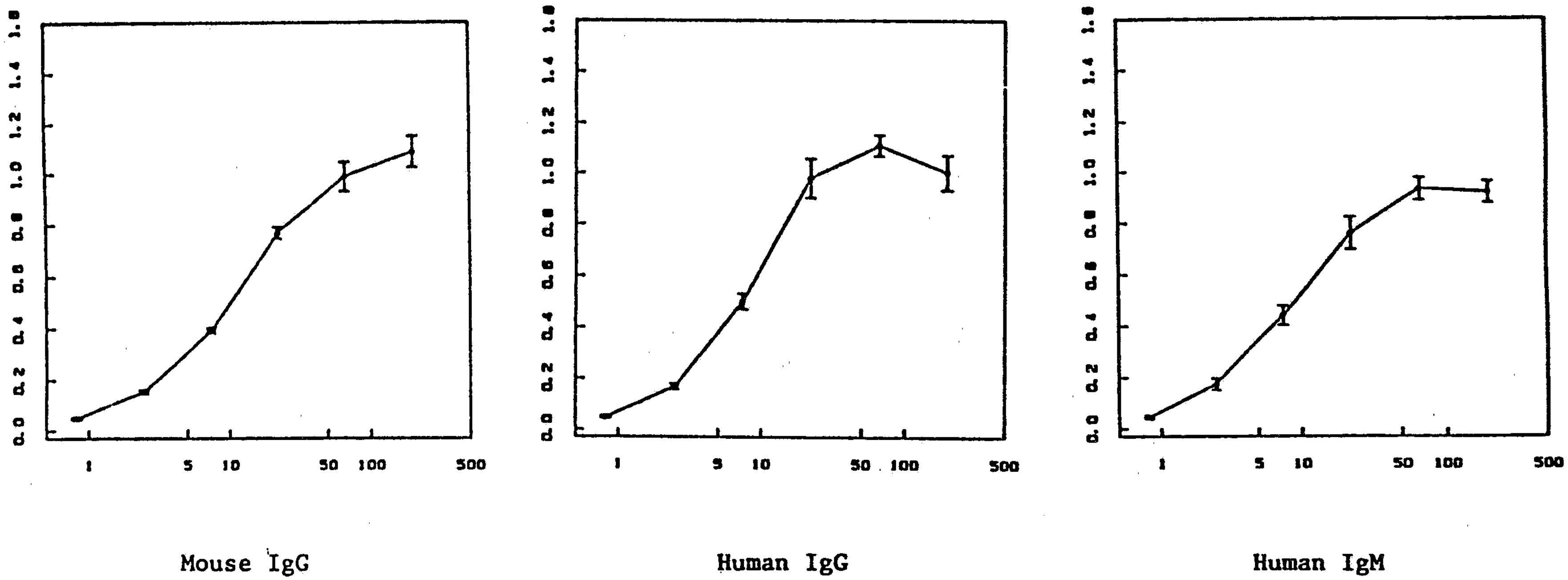

3|3

0164889

FIGURE 3